# EUROPÄISCHE PATENTSCHRIFT

(11) **EP 1 792 577 B1**
(45) Veröffentlichungstag und Bekanntmachung des Hinweises auf die Patenterteilung: **19.05.2010**
(21) Anmeldenummer: 07006072.8
(22) Anmeldetag: 14.10.2004
(51) Int. Cl.: A61B 17/70, A61B 17/64

(54) **Chirurgische Stabilisierungseinrichtung mit einem stabförmigen Element**
Surgical stabilisation device with a rod-shaped element
Stabilisateur chirurgical avec un élément en forme de tige

(30) Priorität: 17.10.2003 DE 10348329; 17.10.2003 US 512113 P
(43) Veröffentlichungstag der Anmeldung: 06.06.2007
(62) Teilanmeldung aus: 04024539.1
(73) Patentinhaber: BIEDERMANN MOTECH GmbH, 78054 Villingen-Schwenningen (DE)
(72) Erfinder: Harms, Jürgen, 76227 Karlsruhe (DE); Biedermann, Lutz, 78048 VS-Villingen (DE); Matthis, Wilfried, 79367 Weisweil (DE)
(74) Vertreter: Hofer, Dorothea

(56) Entgegenhaltungen:
- WO-A-02/07621
- WO-A-96/15729
- WO-A-96/16608
- WO-A-2004/105577
- FR-A- 2 702 361
- FR-A- 2 717 370
- US-A- 6 162 223
- US-A1- 2003 191 470
- US-A1- 2003 220 643

## Beschreibung

Die Erfindung betrifft eine Stabilisierungseinrichtung mit einem stabförmigen Element für die Anwendung in der Wirbelsäulen- oder Unfallchirurgie.

Aus der EP 0 669 109 B1 ist eine Stabilisierungseinrichtung zum Stabilisieren benachbarter Rückenwirbel bekannt, die zwei monoaxiale Pedikelschrauben und ein Band umfasst, welches in den Aufnahmeteilen der Pedikelschrauben jeweils über eine Klemmschraube befestigt ist und welche ein auf das Band aufgezogenes Stützelement in Form eines druckfesten Körpers beinhaltet. Diese Stabilisierungseinrichtung ist jedoch nicht torsionssteif. Die Verwendung von monoaxialen Pedikelschrauben limitiert ferner die Anwendung dieser Stabilisierungseinrichtung. Eine ähnliche Stabilisierungseinrichtung bei der anstelle von monoaxialen Pedikelschrauben polyaxiale Pedikelschrauben verwendet werden ist aus der EP 1 188 416 A1 bekannt.

Aus der US 6,162,223 ist eine Fixationseinrichtung für ein Gelenk, z.B. für ein Handgelenk oder ein Kniegelenk, bekannt, bei der ein an seinen Enden mit Knochenverankerungselementen verbundener Fixationsstab zweiteilig ausgebildet ist, wobei die zwei Teile des Fixationsstabs über ein flexibles Kupplungsteil miteinander verbunden sind und wobei die Fixationsstäbe und das Kupplungsteil außerhalb des Körpers angebracht sind. Die einander zugewandten Enden der zwei Teile des Fixationsstabs sind halbkugelförmig ausgebildet und stoßen aneinander, um so eine Art Gelenk zu simulieren, das in seiner Bewegungsfreiheit durch das flexible Kupplungsteil begrenzt ist. Die bekannte Fixationseinrichtung ist aufgrund ihres komplizierten und voluminösen Aufbaus zum Einsatz an der Wirbelsäule nicht geeignet.

Aus der US 2003/0109880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schrauben verbindenden Feder und eine solche Feder umfasst. Die Feder selbst ist als ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, dass die Feder aufgrund ihrer Elastizität dem Druck der Klemmschraube ausweicht und somit die Fixierung zwischen der Knochenschraube und der Feder gelockert wird.

US 2003/0191470 A1 beschreibt eine dynamische Fixierungsvorrichtung, die es Wirbeln, an denen sie befestigt ist, erlaubt, sich innerhalb der normalen physiologischen Grenzen zu bewegen. Die Vorrichtung weist zwei Knochenverankerungsele-' mente auf und einen flexiblen Abschnitt und zwei Enden, die mit den Knochenverankerungselementen verbunden werden können. Der Oberbegriff des Anspruchs 1 basiert auf dieser Offenbarung.

Die FR 2 717 370 beschreibt eine Zwischenwirbelprothese zur Stabilisierung. Sie besteht aus einem zylindrischen Körper mit einem helixförmigen Schlitz, wobei innerhalb des zylindrischen Körpers ein viskoelastisches Material vorhanden ist. An beiden Enden des zylindrischen Körpers sind Stangen angebracht, die mit Knochenschrauben verbunden werden

Es ist Aufgabe der Erfindung ein stabförmiges Element für die Stabilisierung und Bewegungsbegrenzung von miteinander zu verbindenden Wirbeln oder Knochen bereitzustellen, welches einfach und kompakt gebaut, leicht zu handhaben und vielfältig einsetzbar bei gleichzeitig hoher Sicherheit im Einsatz ist. Ferner ist es Aufgabe der Erfindung, eine dynamische Stabilisierungseinrichtung mit kompakter Bauweise, die ein derartiges stabförmiges Element verwendet, und ein Herstellungsverfahren für das stabförmiges Element bereitzustellen.

Die Aufgabe wird gelöst durch, eine dynamische Stabilisierungseinrichtung nach dem Patentanspruch 1.

Weiterbildungen der Erfindung sind in den Unteransprüchen angegeben.

Die Erfindung weist den Vorteil auf, dass das stabförmige Element axiale Kräfte, Biegekräfte und Torsionskräfte aufnimmt, und mit den bekannten polyaxialen oder monoaxialen Knochenschrauben verwendbar und sicher mit diesen zu fixieren ist. Das stabförmige Element ist insbesondere für den Einsatz bei der Stabilisierung und Bewegungsbegrenzung von aneinandergrenzenden Wirbeln bei Bandscheibendefekten unterschiedlichen Schweregrades geeignet. Diese Eigenschaften sind bei der Herstellung auf einfache Art durch Ändern der Dimension des stabförmigen Elements zu verwirklichen.

Weitere Merkmale und Zweckmäßigkeiten der Erfindung ergeben sich aus der Beschreibung von Ausführungsbeispielen anhand der Figuren. Nur wenn sie einen eingeschobenen Kern nach Anspruch 1 aufweisen sind die Ausführungsbeispiele erfindungsgemäß.

Von den Figuren zeigen:
- Fig. 1: eine schematische perspektivische Ansicht der Stabilisierungseinrichtung mit dem stabförmigen Element in einer ersten Anwendung;
- Fig. 2: eine perspektivische Darstellung des stabförmigen Elements nach einer ersten Ausführungsform;
- Fig. 3a: eine Seitenansicht des stabförmigen Elements nach der ersten Ausführungsform;
- FIG. 3b: eine Schnittansicht des stabförmigen Elements nach der ersten Ausführungsform;
- Fig. 4a: eine perspektivische Ansicht der Verbindung zwischen dem stabförmigen Element und Knochenverankerungselementen;
- Fig 4b: eine Schnittansicht der Verbindung zwischen dem stabförmigen Element und Knochenverankerungselementen;
- Fig. 5: eine Seitenansicht des stabförmigen Elements nach einer zweiten Ausführungsform;
- Fig. 6: eine Seitenansicht des stabförmigen Elements nach einer dritten Ausführungsform;
- Fig. 7: eine um 90° gedrehte Seitenansicht des stabförmigen Elements von Fig. 6 nach der dritten Ausführungsform;
- Fig. 8: eine perspektivische Darstellung des stabförmigen Elements nach einer vierten Ausführungsform;
- Fig. 9: eine Seitenansicht des stabförmigen Elements gemäß Fig. 8;
- Fig. 10: eine Schnittansicht des stabförmigen Elements nach einer fünften Ausführungsform;
- Fig. 11: den Betrieb der erfindungsgemäßen Stabilisierungseinrichtung mit dem stabförmigen Element nach der vierten Ausführungsform in einem ersten Zustand;
- Fig. 12: eine Seitenansicht des stabförmigen Elements gemäß Fig. 11 in dem ersten Zustand;
- Fig. 13: eine perspektivische Ansicht der erfindungsgemäßen Stabilisierungseinrichtung mit dem stabförmigen Element nach der vierten Ausführungsform in einem zweiten Zustand;
- Fig. 14: eine Seitenansicht des stabförmigen Elements von Fig. 13 in dem zweiten Zustand;
- Fig. 15: ein zweites Anwendungsbeispiel der Stabilisierungseinrichtung;
- Fig. 16: ein drittes Anwendungsbeispiel der Stabilisierungseinrichtung;
- Fig. 17: ein viertes Anwendungseispiel der Stabilisierungseinrichtung;
- Fig. 18a: eine perspektivische Ansicht einer fünften Ausführungsform des stabförmigen Elements;
- Fig. 18b: eine Seitenansicht der fünften Ausführungsform mit gestrichelt dargestellten verdeckten Linien;
- Fig. 18c: eine zu Fig. 18b um 90° gedrehte Seitenansicht der fünften Ausführungsform;
- Fig. 18d: einen Schnitt der fünften Ausführungsform senkrecht zu der Kernachse im elastischen Abschnitt;
- Fig. 19a: eine perspektivische Ansicht einer sechsten Ausführungsform des stabförmigen Elements;
- Fig. 19b: eine Seitenansicht der sechsten Ausführungsform;
- Fig. 19c: einen Schnitt der sechsten Ausführungsform entlang Stabachse;
- Fig. 20a: eine Darstellung des unteren Bereichs von Fig. 19c; und
- Fig. 20b: eine perspektivische Darstellung des Kopfteils 134 aus Fig. 20a

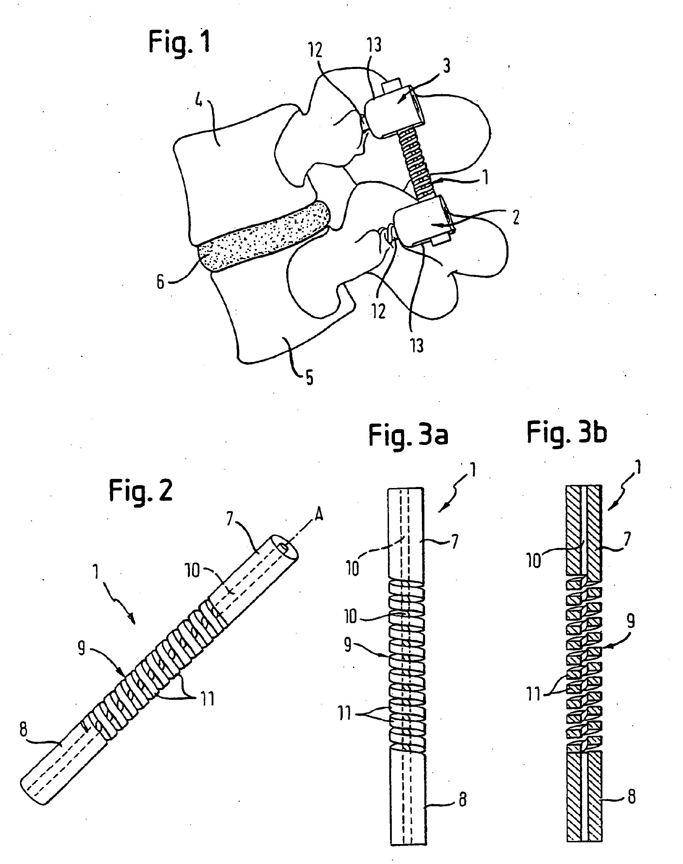

Wie aus Fig. 1 ersichtlich ist, umfaßt die Stabilisierungseinrichtung in einer ersten Anwendung ein stabförmiges Element 1, und zwei Pedikelschrauben 2, 3, die über das stabförmige Element miteinander verbunden sind. Die Pedikelschrauben 2, 3 sind in den Pedikeln zweier aneinandergrenzender Wirbel 4, 5, zwischen denen sich eine geschädigte Bandscheibe 6 befindet, verankert.

Das erfindungsgemäße stabförmige Element 1 ist einstückig ausgebildet. Nach einer ersten Ausführungsform weist es, wie in Fig. 2, 3a und 3b gezeigt ist, einen sich von seinem ersten Ende über eine vorbestimmte Länge erstreckenden ersten starren Abschnitt 7 und einen sich von seinem zweiten Ende über eine vorbestimmte Länge erstreckenden zweiten starren Abschnitt 8 sowie einen zwischen den starren Abschnitten 7, 8 vorgesehenen elastischen Abschnitt 9 von vorbestimmter Länge auf, wobei alle Abschnitte denselben Außendurchmesser haben. Durch das stabförmige Element erstreckt sich ferner eine koaxiale Bohrung 10 mit vorbestimmtem Durchmesser. Der elastische Abschnitt 9 ist als Schraubenfeder mit Windungen 11 mit einer vorbestimmten Steigung ausgebildet. Die Höhe der Windungen 11 des elastischen Abschnitts 9 in Richtung der Längsachse A des stabförmigen Elements, der Durchmesser der koaxialen Bohrung 10, der die Dicke der Windungen 11 in radialer Richtung bestimmt, sowie die Steigung sind so gewählt, dass eine gewünschte Steifigkeit gegenüber axialen Kräften, Biegekräften und Torsionskräften, die auf das stabförmige Element 1 wirken, erzielbar ist.

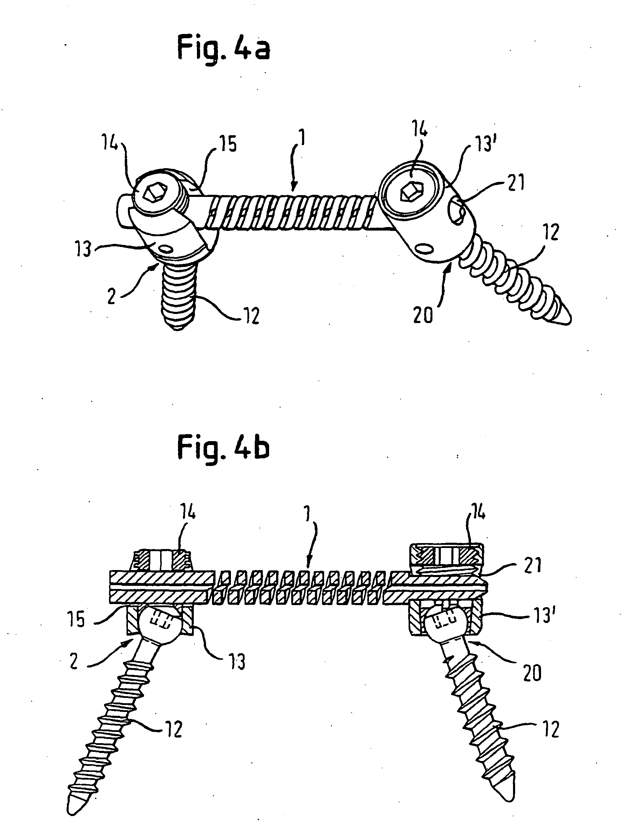

Wie aus Fig. 1, Fig. 4a und Fig. 4b ersichtlich ist, ist weist eine Pedikelschraube 2, 3 der Stabilisierungseinrichtung in bekannter Weise einen Gewindeschaft 12 mit einem Knochengewinde und ein im wesentlichen zylindrisches Aufnahmeteil 13 mit einer U-förmigen Ausnehmung 15 zum Einlegen des stabförmigen Elements auf. Zur Fixierung der starren Abschnitte 7, 8 in dem Aufnahmeteil 13 sind in bekannter Weise in das Aufnahmeteil einschraubbare Innenschrauben 14 vorgesehen. Bevorzugt sind die Pedikelschrauben als Polyaxialschrauben ausgebildet. Die axiale Länge und der Durchmesser der starren Abschnitte 7, 8 des stabförmigen Elements 1 ist so bemessen, dass das stabförmige Element 1 mit seinen starren Abschnitten 7, 8 mit den Pedikelschrauben 2, 3 verbindbar ist. Die Länge der starren Abschnitte 7, 8 entspricht somit wenigstens etwa dem Durchmesser der Innenschraube, die zum Fixieren des stabförmigen Elements vorgesehen ist. Bei Aufnahmeteilen 13' einer Pedikelschraube 20, in die das stabförmige Element nicht von oben eingelegt wird, sondern seitlich in eine Öffnung 21 eingeschoben wird, ist die Länge des starren Abschnitts ebenfalls wenigstens etwa dem Durchmesser eines Fixierelements 14, das das stabförmige Element im Aufnahmeteil 13' fixiert.

In dem in Fig. 1 dargestellten Beispiel der Stabilisierungseinrichtung ist die Länge des elastischen Abschnitts 9 des stabförmigen Elements 1 so gewählt, daß sie im wesentlichen dem Abstand zwischen den Pedikelschrauben 2, 3 im unbelasteten Zustand der Bandscheibe 6 entspricht. Der elastische Abschnitt 9 kann jedoch auch kürzer oder länger sein.

Das stabförmige Element 1 ist aus einem biokompatiblen Material, wie beispielsweise Titan oder einem biokompatiblen Kunststoff, der jedoch keine bzw. geringe Elastomereigenschaften aufweist, gebildet.

Im Betrieb werden zuerst die Pedikelschrauben 2, 3, 20 in die Pedikel aneinandergrenzender Wirbel eingeschraubt und anschlie-ßend das stabförmige Element 1 mit seinen starren Abschnitten 7, 8 jeweils in eines der Aufnahmeteile 14 der Pedikelschrauben 2, 3, 20 eingelegt. Nach der der Positionierung der Wirbel 4, 5 zueinander und der Justierung der Pedikelschrauben 2, 3, 20 relativ zu dem stabförmigen Element werden die starren Abschnitte 7, 8 in den Aufnahmeteilen 13, 13' fixiert. Die Positionierung der Wirbel 4, 5 zueinander erfolgt in einer Anwendung so, dass sich der elastische Abschnitt 9 des stabförmigen Elements 1 in unbelasteten Zustand der Bandscheibe 6 in Ruhestellung befindet. Bei Belastung wirken über die Wirbel und den Bandapparat Kräfte auf die Bandscheibe 6. Das stabförmige Element 1 limitiert über den elastischen Abschnitt 9 die mehrachsige Bewegung der Wirbel relativ zueinander und verhindert so das Einwirken von übermäßig großen Kräften auf die Bandscheibe. Damit kann der Degenerationsprozeß einer geringfügig oder mäßig defekten Bandscheibe aufgehalten werden. Alternativ wird je nach Indikation bereits im unbelasteten Zustand der Wirbelsäule über die Stabilisierungseinrichtung eine vorbestimmte Distraktion der Wirbel vorgenommen, um so die Bandscheibe zu entlasten. Alternativ können Knochenschrauben seitlich direkt in den Wirbelkörpern verankert werden.

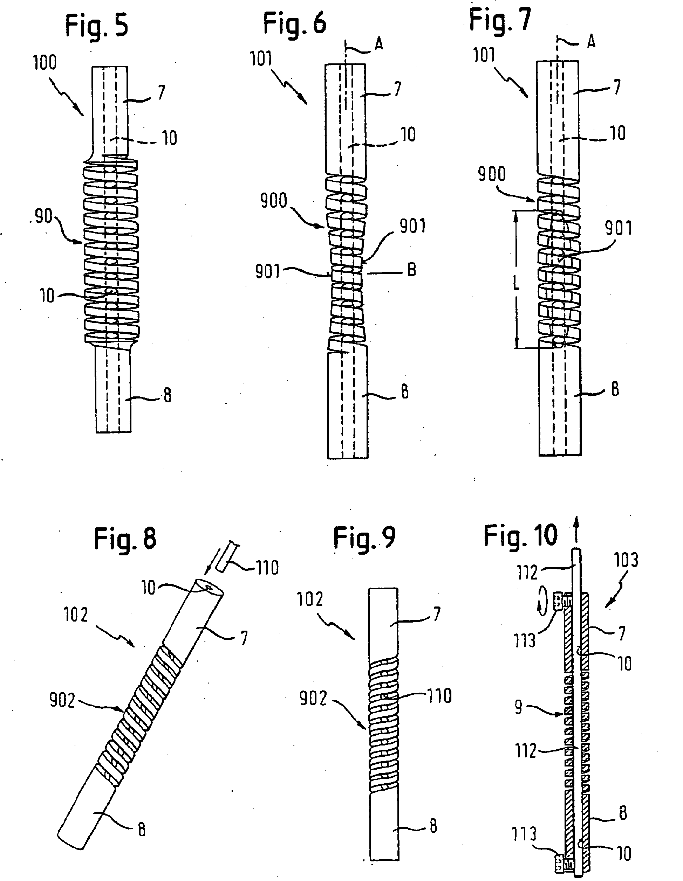

In dem in Fig. 5 gezeigten zweiten Ausführungsbeispiel weist ein stabförmiges Element 100 wie bei der ersten Ausführungsform die starren Abschnitte 7, 8 sowie einen mit diesen einstückig verbundenen zwischen den starren Abschnitten 7, 8 liegenden elastischen Abschnitt 90 in Form einer Schraubenfeder auf. Der Unterschied zur ersten Ausführungsform besteht darin, dass der Durchmesser des elastischen Abschnitts 90 größer ist, als der Durchmesser der starren Abschnitte 7, 8. Dadurch wird eine höhere Steifigkeit im Vergleich zu der Steifigkeit des stabförmigen Elements gemäß der ersten Ausführungsform erzielt. Der Betrieb ist wie bei der ersten Ausführungsform.

In den Figuren 6 und 7 ist ein stabförmiges Element 101 nach einer dritten Ausführungsform dargestellt. Dieses unterscheidet sich von den stabförmigen Elementen 1, 100 der vorhergehenden Ausführungsformen dadurch, dass der zwischen den starren Abschnitten 7, 8 vorgesehene elastische Abschnitt 900 zwei um 180 ° gegeneinander versetzte konkav zur Stabachse hin angeformte Bereiche 901 aufweist. Die Länge L der Bereiche 901 in Richtung der Stabachse ist maximal gleich der Länge des elastischen Abschnitts 901 und der Krümmungsradius ist derart, daß die Windungen der Schraubenfeder nicht durchbrochen sind. Durch diese Ausbildung ist der elastische Abschnitt 900 in einer Richtung B senkrecht zur Stabachse A tailliert ausgebildet und hat somit eine geringere Steifigkeit in dieser Richtung. Damit ist eine orientierte Steifigkeit gegeben, die für bestimmte Anwendungen zweckmäßig ist.

Der Betrieb erfolgt wie bei der ersten und zweiten Ausführungsform mit dem einzigen Unterschied, daß das stabförmige Element 101 in Umfangsrichtung orientiert in den Pedikelschrauben befestigt werden kann. Durch Wählen der Dimensionen des Federabschnitts kann eine gewünschte Steifigkeit präzise ausgewählt und eingestellt werden.

In einer vierten, in den Figuren 8 und 9 gezeigten Ausführungsform weist das stabförmige Element 102 einen sich durch den elastischen Abschnitt 902 koaxial hindurch erstreckenden zylindrischen Kern 110 auf, der eine bestimmte Biegeelastizität hat. Der Durchmesser des Kerns 110 ist so bemessen, daß der Kern 110 nach Einschieben in die Bohrung 10 paßgenau in dieser gehalten ist. Der Kern ist vorzugsweise aus demselben Material wie das stabförmige Element, er kann aber auch aus einem flexiblen Kunststoff bestehen.

In einer Abwandlung ist der Kern 110 einstückig mit den starren Abschnitten 7, 8 und mit den Windungen der Schraubenfeder des elastischen Abschnitts 902 verbunden.

Der Kern 110 bewirkt eine höhere Biegesteifigkeit des stabförmigen Elements 102 verglichen mit der ersten Ausführungsform. So kann bei dieser Ausführungsform eine Steifigkeit ähnlich der des stabförmigen Elements 100 der zweiten Ausführungsform, welches den größeren Durchmesser des elastischen Abschnitts aufweist, erzielt werden. Die Biegesteifigkeit ist ferner durch Wahl des Durchmessers und/oder des Materials des Kerns anpaßbar. Beispielsweise kann auch eine Formgedächtnislegierung mit der bekannten Eigenschaft der Superelastizität verwendet werden.

Der Betrieb erfolgt wie bei den hervorgehenden Ausführungsformen. Im Unterschied zu den vorherigen Ausführungsformen ist jedoch eine Komprimierung bzw. Extension des elastischen Abschnitts 902 in axialer Richtung, sowie eine Torsion dimensionsmäßig reduziert. Es sind dann vorzugsweise nur Flexionsbewegungen zugelassen, was für bestimmte Anwendungen von Vorteil ist.

In einer fünften, in Fig. 10 gezeigten Ausführungsform weist das stabförmige Element 103 die starren Abschnitte 7, 8 und den elastischen Abschnitt 9 wie bei der ersten Ausführungsform auf.

In der vorzugsweise koaxialen Bohrung 10 ist ein Zugelement 112, beispielsweise ein Draht, vorgesehen, das über Fixierelemente, wie z.B. Klemmschrauben 13, an den starren Abschnitten 7, 8 befestigt ist. Im Betrieb kann somit eine Vorspannung des elastischen Abschnitts 9 erzeugt werden.

Die Merkmale der beschriebenen Ausführungsformen sind miteinander kombinierbar. So kann beispielsweise auch das stabförmige Element der zweiten Ausführungsform einen Kern und/oder angeformte Abschnitte zur Erzielung einer orientierten Steifigkeit haben. In einer Abwandlung der dritten Ausführungsform ist der elastische Abschnitt gleichmäßig an einer Stelle tailliert ausgebildet oder es sind mehrere in Umfangsrichtung gleichmäßig beabstandete konkav angeformte Bereiche vorgesehen, um in eine bestimmte Steifigkeit in definierten Richtungen zu erhalten.

In einer weiteren Ausführungsform weist der Stab mehrere starre Abschnitte mit mehreren jeweils dazwischen liegenden elastischen Abschnitten auf, so dass eine Mehrzahl von Pedikelschrauben auf diese Weise miteinander teils starr, teils elastisch verbunden werden kann.

In einer weiteren Ausführungsform ist um den elastischen Abschnitt eine Beschichtung oder eine Schutzhülle aus einem biokompatiblen Material vorgesehen, damit kein Gewebe oder Blutgefäße oder sonstiges Körpermaterial zwischen die Windungen gelangen kann und dadurch verletzt werden kann oder die Funktion des stabförmigen Elements beeinträchtigen kann.

In einer weiteren Ausführungsform sind anstelle von Polyaxialschrauben Monoaxialschrauben vorgesehen oder es wird für die Stabilisierungseinrichtung eine Kombination aus einer Polyaxialschraube und einer Monoaxialschraube oder Kombinationen mehrerer dieser Schrauben verwendet. Auch die Verwendung von Haken anstelle von Knochenschrauben ist denkbar. In einer weiteren Ausführungsform sind die starren Abschnitte und/oder der elastische Abschnitt gekrümmt.

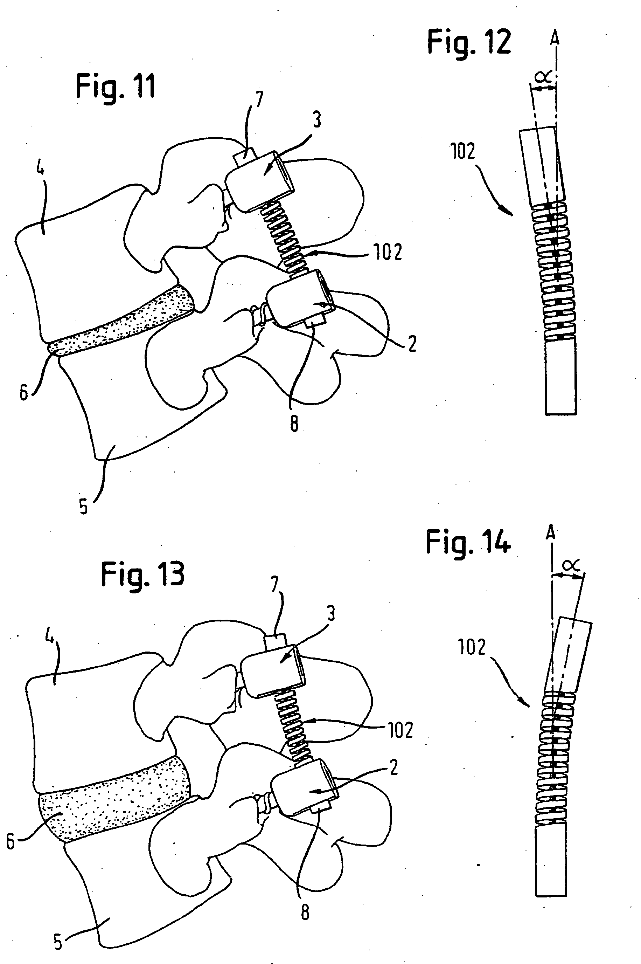

Die Figuren 11 bis 17 zeigen bevorzugte Anwendungen der erfindungsgemäßen Stabilisierungseinrichtung mit dem stabförmigen Element. Bei der Stabilisierungseinrichtung nach den Figuren 11 bis 14 ist das stabförmige Element nach der vierten Ausführungsform verwendet, das den Kern 110 besitzt. Die Stabilisierungseinrichtung wird zum Beispiel eingesetzt, wenn eine geringfügig oder mäßig defekte Bandscheibe 6 unterstützt werden soll und ein Einwirken von schädlichen Kräften auf die Bandscheibe durch Bewegungslimitierung der Wirbel vermieden werden soll. Das stabförmige Element 102 ist starr in axialer Richtung und erlaubt weder eine Kompression noch eine Extension in axialer Richtung. Flexionsbewegungen unter einem Winkel α zur Stabachse, der beispielsweise bis zu ± 8° beträgt, sind jedoch möglich.

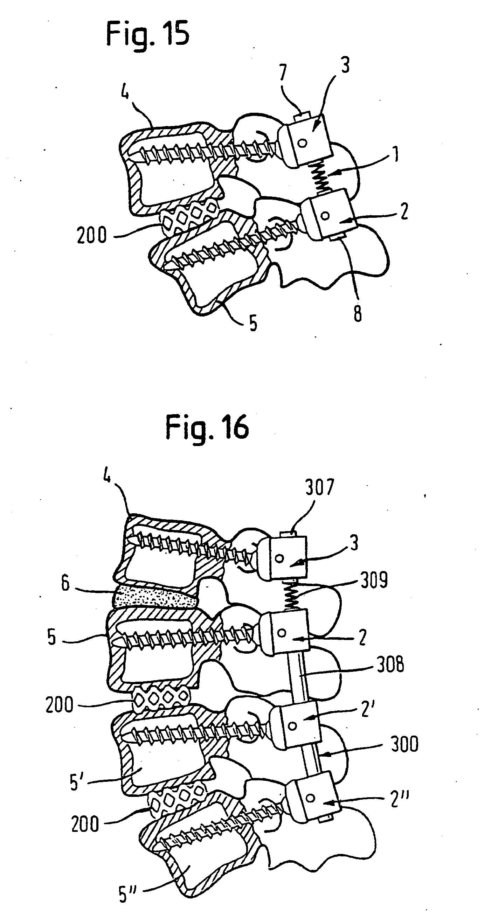

Fig. 15 zeigt die Anwendung der Stabilisierungseinrichtung mit dem stabförmigen Element bei einer Fusion zweier Wirbel 4, 5 mittels eines starren Elements 200, beispielsweise eines Titanzylinders, nach Entfernung der natürlichen Bandscheibe. Hier ist eine höhere Steifigkeit des Stabs erwünscht, um eine ausreichende Bewegungslimitierung zu erreichen. Die geringfügige Bewegungsmöglichkeit der Wirbel zueinander ist jedoch im Vergleich zu einer ausschließlich steifen Verbindung von Vorteil, weil durch die erhöhte zyklische Teilbelastung das Knochenwachstum angeregt wird und die Verknöcherung somit schneller voranschreitet.

Fig. 16 zeigt die Anwendung der dynamischen Stabilisierungseinrichtung als flexibles Ende einer langstreckigen Fusion, bei der mehrere, im gezeigten Beispiel drei Wirbel 5, 5', 5" miteinander über steife Elemente 200 fusioniert werden und posterior über einen starren Stab 300 verbunden sind. Die an den letzten Wirbel 5 der fusionierten Kette angrenzende natürliche Bandscheibe 6, sowie der nächste Wirbel 4 unterliegen überdurchschnittlichen Belastungen, die zu einem höheren Verschleiß der Bandscheibe 6 führen. Um dieses Nachbarsegment vor ungewöhnlichen Bewegungen und damit erhöhten Belastungen zu schützen, wird die Stabilisierungseinrichtung als Bewegungslimitierung vorgesehen. Der Stab 300 weist in diesem Ausführungsbeispiel einen starren Abschnitt 308 auf, der so groß bemessen ist, dass damit drei Pedikelschrauben 2, 2', 2" verbunden werden können, daran angrenzend ist der elastische Abschnitt 309 vorgesehen und am Ende wiederum ein starrer Abschnitt 307 zum Verbinden mit der Pedikelschraube 3.

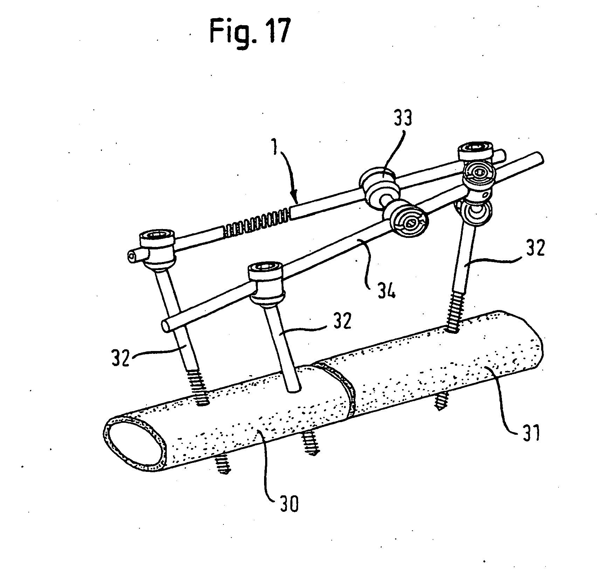

Fig. 17 zeigt die Verwendung des stabförmigen Elements 1 in einer Stabilisierungseinrichtung gemäß einem Fixateur externe zur Stabilisierung von z.B. Röhrenknochen. Knochenteile 30, 31 werden über Knochenschrauben 32, die z.B. über ein Verbindungselement 33 mit einem starren Stab 34 und einem erfindungsgemäßen stabförmigen Element 1 verbunden sind, stabilisiert.

Bei einem Herstellungsverfahren für das stabförmige Element wird in einem ersten Schritt ein starrer Stab eines gewünschten Durchmessers aus einem körperverträglichen Material, wie beispielsweise Titan bereitgestellt. Anschließend wird an einem Abschnitt zwischen den Enden des Stabes mittels Fräsen der elastische Abschnitt 9, 900, 902 in Form einer Schraubenfeder erzeugt. Der durch den Federabschnitt hindurchgehende Kern 110 wird anschließend, falls gewünscht, herausgebohrt, womit der Stab nach der ersten Ausführungsform hergestellt ist.

Zum Erzeugen des Stabes nach der vierten Ausführungsform wird der Kern 110 entweder belassen oder ein separater Kern nachträglich eingeschoben.

Zum Erzeugen des Stabes nach der zweiten Ausführungsform wird als Ausgangsmaterial ein Stab mit einem Durchmesser, der dem Durchmesser des gewünschten elastischen Abschnitts 90 entspricht, bereitgestellt. Anschließend wird die Schraubenfeder durch Fräsen erzeugt. Dann werden die starren Endabschnitte 7, 8 auf den gewünschten Durchmesser abgedreht.

Zum Herstellen des Stabes nach der dritten Ausführungsform wird an Stellen des elastischen Abschnitts, die um 180° in Umfangsrichtung voneinander versetzt sind, in einem Bereich Materialentfernt, um so eine orientierte Taillierung zu erzeugen.

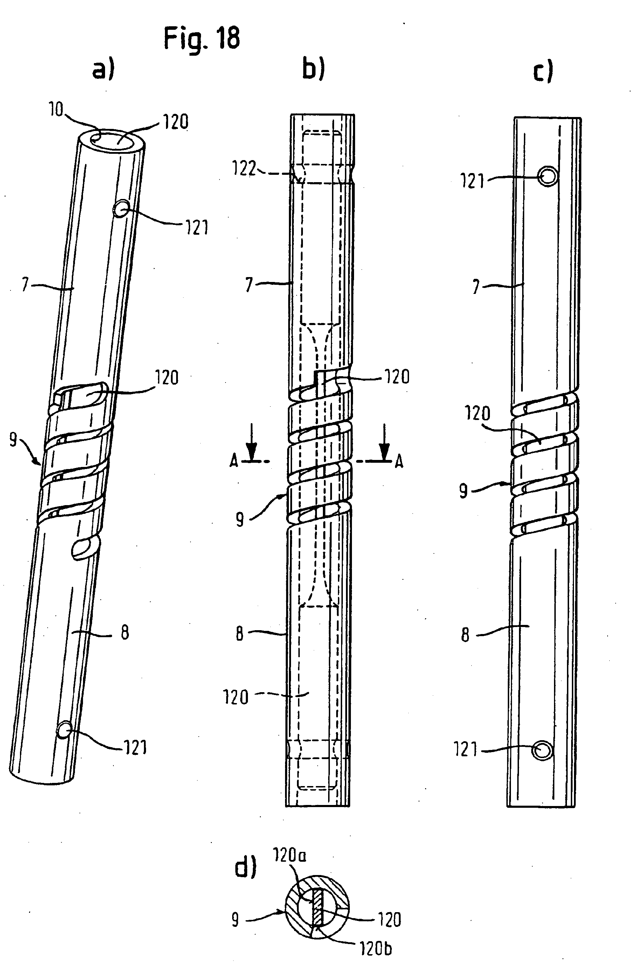

Ein weiteres Ausführungsbeispiel ist in den Fig. 18a bis 18d dargestellt. Bei dieser Ausführungsform ist der Kern 120 derart ausgebildet, dass er zumindest in einem Teil des elastischen Abschnitts 9 in der Ebene senkrecht zu der Stabachse einen rechteckigen Querschnitt aufweist (siehe Fig. 18). Der rechteckige Querschnitt ist dabei, wie in Fig. 18d gezeigt ist, mit einer langen Seite 120a und einer kurzen Seite 120b ausgebildet. Wie in Fig. 18b dargestellt ist, weist der Kern 120 dabei vorzugsweise in den Bereichen, die sich bei eingesetztem Kern 120 in den starren Abschnitten 7, 8 befinden einen dem Innendurchmesser der Bohrung 10 angepassten runden Querschnitt auf, sodass eine Befestigung des Stabes wie bei den vorgenannten Ausführungsformen gewährleistet ist. Die Befestigung kann zum Beispiel durch einen sich durch eine querlaufende Bohrung 122 erstreckenden Stift erreicht werden.

Durch die rechteckige Ausführung des Kernes 120 in dem Bereich des elastischen Abschnitts 9 ist eine große Biegesteifigkeit in der Richtung der langen Seite 120a des rechteckigen Querschnitts und eine geringere Biegesteifigkeit in der Richtung der kurzen Seite 120b des rechteckigen Querschnitts erzeugt. Je nach Ausrichtung des Kernes 120 kann somit für bestimmte Anwendungen eine erhöhte Beweglichkeit in einer Richtung und eine im Verhältnis dazu eingeschränkte Beweglichkeit in der Richtung senkrecht zu dieser ermöglicht werden. Verglichen mit den Ausführungsformen, bei denen kein Kern bzw. ein Draht in der Bohrung vorgesehen sind, ist dabei die Zug- und Druckfestigkeit erhöht. Ferner kann die Torsionssteifigkeit durch Auswahl eines geeigneten Kerns eingestellt werden.

Um den beschriebenen Effekt zu erzielen, muss der Abschnitt des Kernes nicht zwangsläufig einen rechteckigen Querschnitt aufweisen, sondern auch andere Querschnittsformen, wie z.B. ein ovaler Querschnitt, ein im Wesentlichen rechteckiger Querschnitt mit teilweise konkav oder konvex geformten Seiten oder sogar ein im Wesentlichen dreieckiger Querschnitt können je nach gewünschten Eigenschaften gewählt werden. Wichtig ist, dass der Querschnitt in zwei zueinander senkrechten Achsen (oder zumindest in zwei unterschiedlichen Richtungen) in der Ebene senkrecht zur Stabachse unterschiedliche Abmessungen aufweist. Hierdurch werden richtungsabhängige Biegesteifigkeiten erzielt.

Der Kern 120 kann bei dieser Ausführungsform wie bei den anderen Ausführungsformen ( von der Geometrie abhängig) wieder entweder einstückig mit dem Stab ausgebildet oder separat ausgebildet und in diesen eingesetzt sein. Eine Kombination dieses Kernes 120 mit den anderen Ausführungsformen ist möglich, um die Eigenschaften des stabförmigen Elementes den jeweiligen Anforderungen anzupassen.

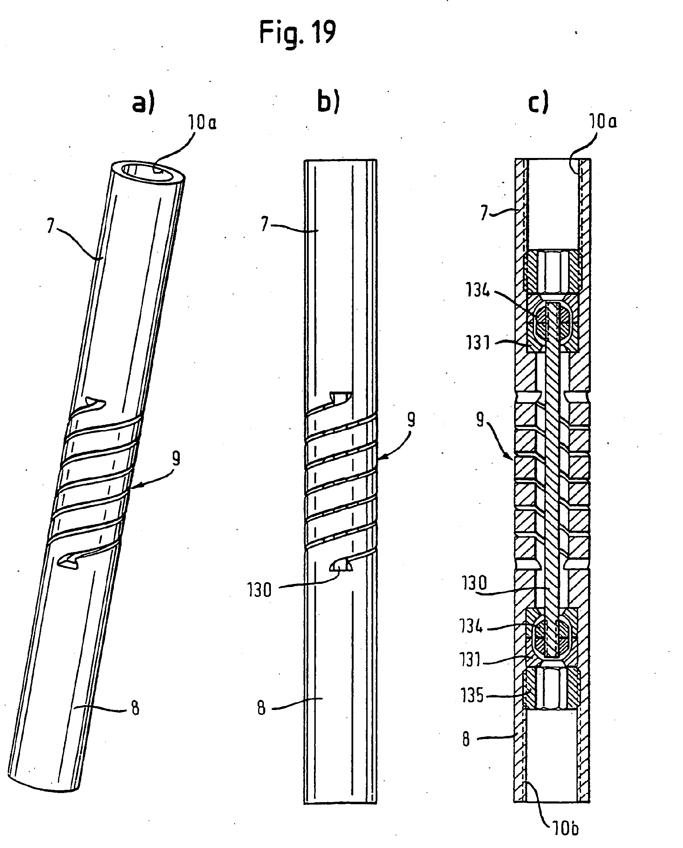

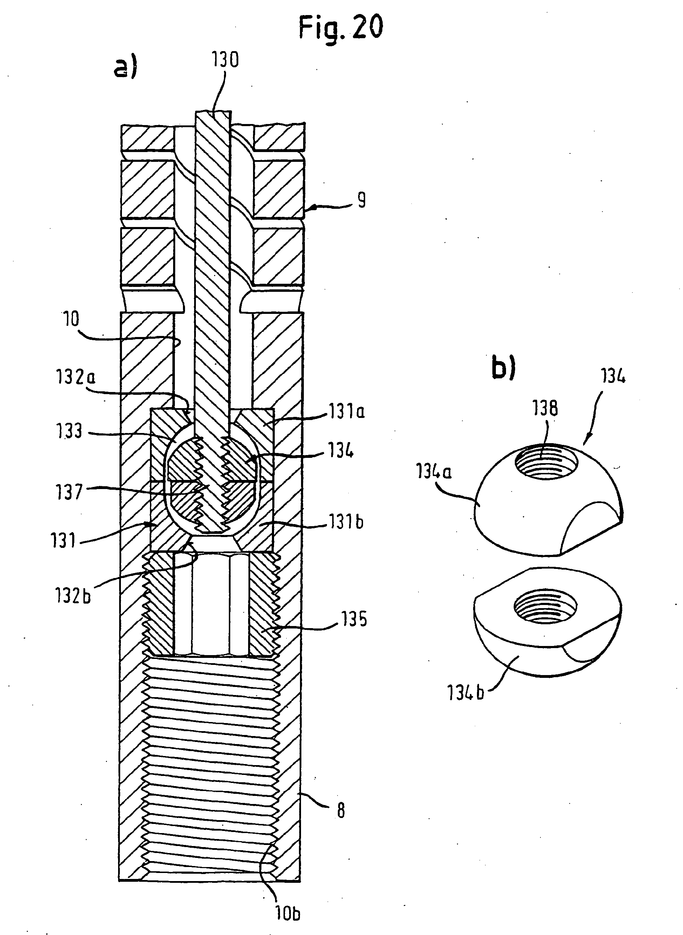

Bei der in den Fig. 19a bis 19c und Fig. 20a dargestellten Ausführungsform ist eine spezielle Befestigung für den Kern vorgesehen. Wie in Fig. 19c dargestellt ist, schließt sich in den starren Abschnitten 7, 8 jeweils in Richtung des freien Endes des stabförmigen Elements an die Bohrung 10 eine koaxial zu dieser angeordnete zweite Bohrung 10a, 10b an. Bei der dargestellten Ausführungsform sind die starren Abschnitte 7, 8 auf beiden Seiten des elastischen Abschnitts 9 symmetrisch ausgebildet, weshalb im Folgenden anhand von Fig. 20a nur der Abschnitt 8 beschrieben wird.

In der zweiten Bohrung 10b ist angrenzend an die Bohrung 10 ein Kopfaufnahmeteil 131 vorgesehen, das in Richtung senkrecht zu der Achse der zweiten Bohrung 10b einen Außendurchmesser aufweist, der im Wesentlichen dem Innendurchmesser der zweiten Bohrung 10b entspricht. Das Kopfaufnahmeteil besteht aus zwei Schalen 131a, 131b, die in Richtung der Achse der Bohrung 10 angeordnet sind und miteinander einen Hohlraum 133 umschließen, der im Wesentlichen die Form einer in Richtung der Achse der Bohrung 10 gestreckten Kugel aufweist. In den Stirnflächen der Schalen 131a, 131b sind koaxial zu der zweiten Bohrung 10b Bohrungen 132a und 132b vorgesehen, deren Durchmesser von dem Hohlraum 133 zu der jeweiligen Stirnfläche zunimmt und etwas größer ist, als der Durchmesser des Kerns 130.

Der Außendurchmesser des Kerns 130 ist wesentlich kleiner als der Innendurchmesser der Bohrung 10. Das dem starren Abschnitt 8 zugewandte Ende des Kerns 130 ist mit einem Außengewinde 137 versehen, das mit einem Innengewinde 138 in einer Bohrung eines Kopfteils 134 zusammenwirkt. Das Kopfteil 134 ist in Fig. 20b dargestellt. Es besteht aus zwei im Wesentlichen halbkugelförmigen Hälften 134a und 134b die zusammengesetzt eine im Wesentlichen kugelförmige Außenseite mit einem Durchmesser aufweisen, der größer als der kleinste Durchmesser der Bohrung 132b und kleiner als der kleinste Durchmesser des Hohlraums 133 ist. Das mit dem Außengewinde versehene freie Ende des Kerns 130 ist durch die Bohrung 132b geführt und in die in dem Hohlraum 133 angeordneten Hälften 134a und 134b des Kopfteils 134 eingeschraubt, die als Mutter und Kontermutter zusammenwirken.

Das andere freie Ende des Kerns 130 ist in dem starren Abschnitt 7 in ähnlicher Weise aufgenommen. Je nachdem wie weit die Kopfteile 134 auf die beiden freien Enden des Kernes 130 aufgeschraubt sind, liegen die Kopfteile 134 an den Innenseiten der Kopfaufnahmeteile 131 an, die der Bohrung 10 zugewandt sind, liegen an den den freien Enden der zweiten Bohrungen 10a, 10b zugewandten Innenseiten an oder weisen zu diesen Innenseiten jeweils etwas Spiel auf.

Bei dieser Ausführungsform können durch die kugelförmige Ausbildung der Kopfteile 134 in Zusammenwirken mit der im Wesentlichen sphärischen Form der Kopfaufnahmeteile 131 die Kopfteile 134 in den Kopfaufnahmeteilen 131 gleiten. Bei einer einwirkenden externen Biegebelastung ist dadurch eine Biegebelastung des Kerns 130 für kleine Auslenkungen verhindert. Durch Aufschrauben der Kopfteile 134, sodass diese an den kernseitigen Innenseiten der Kopfaufnahmeteile 131 bzw. an den kernabgewandten Innenseiten der Kopfaufnahmeteile 131 anliegen, kann eine Zug- bzw. Druckverstärkung für das stabförmige Element erzielt werden. Durch beidseitiges Spiel der Kopfteile 134 in den Aufnahmeteilen 131 kann eingestellt werden, dass eine Wirkung des Kernes erst ab einer bestimmten Auslängung bzw. Verkürzung des Außenbereichs des stabförmigen Elements eintritt.

In den Fig. 19 bis 20a ist beispielhaft dargestellt, dass die Aufnahmeteile 131 mit Schrauben 135 in den zweiten Bohrungen 10a, 10b befestigt sind; es ist jedoch zum Beispiel ebenfalls möglich, zur Befestigung seitliche Schrauben wie bei anderen Ausführungsbeispielen oder Pressverbindung zwischen den Kopfaufnahmeteilen und den zweiten Bohrungen vorzusehen.

Weiterhin sind Kombinationen von Teilen dieser Ausführungsformen mit anderen Ausführungsformen möglich.

## Patentansprüche

1. Stabilisierungseinrichtung für Knochen mit wenigstens zwei Knochenverankerungselementen (2, 3) mit jeweils einem Knochenverankerungsabschnitt (12) zum Verankern im Knochen und einem Aufnahmeteil (13) und mit einem, mit den Knochenverankerungselementen zu verbindenden stabförmigen Element (1, 100, 101, 102, 103, 300),
wobei das stabförmige Element wenigstens einen starren Abschnitt (7, 8, 307, 308) aufweist, der so dimensioniert ist, daß er in dem Aufnahmeteil (13) aufnehmbar ist, und angrenzend an den starren Abschnitt einen elastischen Abschnitt (9, 90, 900, 902, 309) aufweist, wobei der starre Abschnitt und der elastische Abschnitt aus einem Stück ausgebildet sind **dadurch gekennzeichnet, daß**, der elastische Abschnitt (9, 90, 900, 902, 309) durch eine helixförmige schlitzförmige Ausnehmung in der äußeren Oberfläche des stabförmigen Elements gebildet ist, die sich radial in Richtung der Stabmittenachse erstreckt,
wobei ferner das Aufnahmeteil im wesentlichen zylindrisch mit einer U-förmigen Ausnehmung (15) zum Einlegen des stabförmigen Elements ausgebildet ist, eine in das Aufnahmeteil einschraubbare Innenschraube (14) vorgesehen ist und die Länge des starren Abschnitts (7, 8) wenigstens etwa dem Durchmesser der Innenschraube, die zum Fixieren des stabförmigen Elements vorgesehen ist, entspricht, und
eine sich durch das stabförmige Element erstreckende vorzugsweise koaxiale Bohrung (10) vorgesehen ist, wobei
der elastische Abschnitt (902) einen Kern (110, 112, 120) aufweist, der nach Einschieben in die Bohrung (10) passgenau in dieser gehalten ist.

2. Stabilisierungseinrichtung nach Anspruch 1, wobei der elastische Abschnitt (9, 90, 900, 902, 309) als Schraubenfeder ausgebildet ist.

3. Stabilisierungseinrichtung nach Anspruch 1 oder 2, **dadurch gekennzeichnet, dass** angrenzend an das dem starren Abschnitt (7, 307) gegenüberliegende Ende des elastischen Abschnitts (9, 90, 901, 902, 309) ein zweiter starrer Abschnitt (8, 308) vorgesehen ist.

4. Stabilisierungseinrichtung nach einem der Ansprüche 1 bis 3, **dadurch gekennzeichnet, dass** der Außendurchmesser des elastischen Abschnitts (90, 90) wenigstens an einer Stelle verschieden von dem Außendurchmesser des starren Abschnitts (7, 8, 307, 308) ist.

5. Stabilisierungseinrichtung nach einem der Ansprüche 1 bis 4, **dadurch gekennzeichnet, daß** der elastische Abschnitt (900, 901) in einer bestimmten Richtung senkrecht zur Stabachse wenigstens streckenweise einen kleineren oder größeren Außendurchmesser hat, als in einer anderen Richtung.

6. Stabilisierungseinrichtung nach einem der Ansprüche 1 bis 5 , **dadurch gekennzeichnet, dass** der Außendurchmesser des elastischen Abschnitts (900, 901) über die Länge des elastischen Abschnitts variiert.

7. Stabilisierungseinrichtung für Knochen nach einem der Ansprüche 1 bis 6, **dadurch gekennzeichnet, dass** das Knochenverankerungselement (2, 3, 20) eine Monoaxial- oder eine Polyaxial-Knochenschraube ist.

8. Stabilisierungseinrichtung nach einem der Ansprüche 1 bis 7, **dadurch gekennzeichnet, dass** der Kern (120) zumindest in einem Teil des elastischen Abschnittes mit einem anisotropen Querschnitt ausgebildet ist.

9. Stabilisierungseinrichtung nach einem der Ansprüche 1 bis 8, **dadurch gekennzeichnet, dass** der Kern (120) in dem stabförmigen Element mit Spiel in Richtung der Stabachse aufgenommen ist.

## Claims

1. Stabilization apparatus for bones with at least two bone anchoring elements (2, 3), each comprising a bone anchoring section (12) to be anchored in the bone and a receiver member (13), and with a rod-shaped element (1, 100, 101, 102, 103, 300) to be connected to the bone anchoring elements, wherein the rod-shaped element has at least one rigid section (7, 8, 307, 308), which is dimensioned such that it can be placed into the receiver member (13) and further comprising a flexible section (9, 90, 900, 902, 309) adjacent to the rigid section, wherein the rigid section and a flexible section are formed of one piece, **characterized in that** the flexible section (9, 90, 900, 902, 309) is formed by a helical slotted opening in the outer surface of the rod-shaped element, the opening extending radially in the direction of the rod center axis,
and wherein the receiver member is formed substantially cylindrically with a U-shaped recess (15) for inserting the rod-shaped element and an inner screw (14) which is to be screwed into the receiver member is provided and wherein the length of the rigid section (7, 8) corresponds at least to approximately the diameter of the inner screw which is provided for fixing the rod-shaped element.

2. Stabilization apparatus according to claim 1, wherein the flexible section (9, 90, 900, 902, 309) is formed as a helical spring.

3. Stabilization apparatus according to claim 1 or 2, **characterized in that** a second rigid section (8, 308) is provided adjacent to that end of the flexible section (9, 90, 901, 902, 309) that is arranged opposite to the rigid section (7, 307).

4. Stabilization apparatus according to one of claims 1 to 3, wherein the outer diameter of the flexible section (90, 90) is different from the outer diameter of the rigid section (7, 8, 307, 308) at at least one point.

5. Stabilization apparatus according to one of claims 1 to 4, **characterized in that** the flexible section (900, 901) has, at least in parts, a smaller or greater outer diameter in a specific direction that is perpendicular to the axis of the rod than in another direction.

6. Stabilization apparatus according to one of claims 1 to 5, **characterized in that** the outer diameter of the flexible section (900, 901) varies along the length of the flexible section.

7. Stabilization apparatus according to one of claims 1 to 6, **characterized in that** a preferably coaxial bore (10) is provided that extends through the rod-shaped element.

8. Stabilization apparatus according to one of claims 1 to 7, **characterized in that** the flexible section (902) comprises a core (110, 112, 120).

9. Stabilization apparatus for bones according to one of claims 1 to 8, **characterized in that** the bone anchoring element (2, 3, 20) is a monoaxial or a polyaxial bone screw.

10. Stabilization apparatus according to claim 8, **characterized in that** the core (120) is in at least one part of the flexible section formed with an anisotropic cross-section.

11. Stabilization apparatus according to one of claims 8 or 10, **characterized in that** the core (120) is accommodated in the rod-shaped element with a tolerance in the direction of the rod axis.

## Revendications

1. Dispositif de stabilisation pour os comprenant au moins deux éléments d'ancrage osseux (2, 3) comprenant chacun une partie d'ancrage osseux (12) pour l'ancrage dans l'os et une partie de réception (13) et avec un élément (1, 100, 101, 102, 103, 300) en forme tige et à relier avec les éléments d'ancrage osseux,
l'élément en forme de tige comprenant au moins une partie (7, 8, 307, 308) rigide; qui est dimensionnée de telle sorte qu'elle peut être réceptionnée dans la partie de réception (13), et de façon continue à la partie rigide une partie (9, 90, 900, 902, 309) élastique, la partie rigide et la partie élastique étant conçues d'une seule pièce,
**caractérisé en ce que** la partie élastique (9, 90, 900, 902, 309) est formée par un évidement en forme de fente et hélicoïdal dans la surface extérieure de l'élément en forme de tige, qui s'étend radialement en direction de l'axe central de tige,
et, en plus, la partie de réception est formée pour l'essentiel cylindrique avec un évidement (15) en forme de U pour placer l'élément en forme de tige dedans, et une vis intérieure (14) est prévue qui peut être vissée dans la partie de réception et **en ce que** la longueur de la partie rigide (7, 8) correspond au moins approximativement au diamètre de la vis intérieure qui est prévue pour fixer l'élément en forme de tige.

2. Dispositif de stabilisation pour os selon la revendication 1, **caractérisé en ce que** la partie (9, 90, 900, 902, 309) élastique est conçue comme ressort cylindrique.

3. Dispositif de stabilisation selon la revendication 1 ou 2, **caractérisé en ce qu'**une second partie (8, 308) rigide est prévue de façon contiguë à l'extrémité, opposée à la partie (7, 307) rigide, de la partie (9, 90, 901, 902, 309) élastique.

4. Dispositif de stabilisation selon l'une quelconque des revendications 1 à 4, **caractérisé en ce que** le diamètre extérieur de la partie (90, 90) élastique est différent du diamètre extérieur de la partie (7, 8, 307, 308) rigide au moins en un endroit.

5. Dispositif de stabilisation selon l'une quelconque des revendications 1 à 4, **caractérisé en ce que** la partie (900, 901) élastique a dans une direction définie perpendiculairement à l'axe de la tige au moins par tronçon un diamètre extérieur plus petit ou plus grand que dans une autre direction.

6. Dispositif de stabilisation selon l'une quelconque des revendications 1 à 5, **caractérisé en ce que** le diamètre extérieur de la partie (900, 901) élastique varie sur la longueur de la partie élastique.

7. Dispositif de stabilisation selon l'une quelconque des revendications 1 à 6, **caractérisé en ce qu'**un perçage (10), de préférence coaxial, s'étendant à travers l'élément en forme de tige est prévu.

8. Dispositif de stabilisation selon l'une quelconque des revendications 1 à 7, **caractérisé en ce que** la partie élastique (902) présente un noyau (110, 112, 120).

9. Dispositif de stabilisation pour os selon l'une quelconque des revendications 1 à 8, **caractérisé en ce que** l'élément d'ancrage osseux (2, 3, 20) est und vis à os monoaxiale ou und vis à os polyaxiale.

10. Dispositif de stabilisation selon la revendication 8,
**caractérisé en ce que** le noyau (120) est conçu au moins dans un tronçon de la partie élastique avec une section anisotrope.

11. Dispositif de stabilisation selon l'une des revendications 8 ou 10, **caractérisé en ce que** le noyau (120) est réceptionné dans l'élément en forme de tige avec du jeu en direction de l'axe de la tige.
